# EUROPEAN PATENT APPLICATION

(11) **EP 1 925 318 A1**
(43) Date of publication of application: **28.05.2008**
(21) Application number: 06124336.6
(22) Date of filing: 20.11.2006
(51) Int. Cl.: A61K 39/145, A61K 38/00

(54) **Recombinant modified vaccinia virus Ankara (MVA)-based vaccine for the avian flu**

(71) Applicant: Paul-Ehrlich-Institut, 63225 Langen (DE)
(72) Inventor: The designation of the inventor has not yet been filed
(74) Representative: Vossius & Partner

(57) **Abstract**

The present invention relates to the use of a recombinant modified vaccinia virus Ankara (MVA) comprising a heterologous nucleic acid for the production of a medicament in particular a vaccine, wherein the sequence of heterologous nucleic acid is from influenza A virus class H5 antigen. In a further aspect of the invention the invention relates to a recombinant modified vaccinia virus Ankara (MVA) comprising a heterologous nucleic acid, wherein (a) the heterologous nucleic acid is incorporated into a non-essential site within the genome of MVA, (b) the heterologous nucleic acid is under the control of a vaccinia virus promoter, or orthopoxvirus promoter, or poxvirus-specific promoter and, (c) the heterologous nucleic acid is selected from the group of nucleic acids encoding a gene or a part of a gene from an influenza A virus class H5.

## Description

### Introduction

Since the first human cases of H5N1 infections in 1997, influenza viruses of this subtype continue to cause outbreaks of fowlplague worldwide associated with an accumulating number of bird to human transmissions. As of September 14^{th}, 246 human cases were recorded of which 144 proved to be fatal (WHO disease alert 2006. Confirmed human cases of avian influenza H5N1-http://www.who.int/csr/disease/avian_influenza
/country/cases_table_2006_09_14/en/i ndex.html, accessed September 14th, 2006). In addition, the H5N1 virus infections have spread from South-East Asia to other continents of the world (WHO. 2006. Affected areas with confirmed cases of H5N1 avian influenza since 2003, status as of 23.08.2006). Since these viruses not only infect avian species but also various mammalian species (Vahlenkamp, T. W., and T. C. Harder. 2006. Influenza virus infections in mammals. Berl Munch Tierarztl Wochenschr 119:123-31) including humans (Beigel, J. H., J. Farrar, A. M. Han, F. G. Hayden, R. Hyer, M. D. de Jong, S. Lochindarat, T. K. Nguyen, T. H. Nguyen, T. H. Tran, A. Nicoll, S. Touch, and K. Y. Yuen. 2005. Avian influenza A (H5N1) infection in humans. N Engl J Med 353:1374-85) there is a risk of the emerging of a new pandemic strain, either through adaptation of the avian viruses to replication in mammalian species or through the exchange of gene segments with normal epidemic influenza A viruses.

In the light of a pandemic threat caused by influenza H5N1 viruses that are transmitted from infected poultry to human in ever increasing numbers, the availability of sufficient numbers of safe and effective vaccines is considered a priority (WHO. 2006. Antigenic and genetic characteristics of H5N1 viruses and candidate H5N1 vaccine viruses developed for potential use as pre-pandemic vaccines- htttp://who.int/csr/disease/avian_influenza/guidelines/recommendation vaccine.pdf).
However, the development of such vaccines and the production of sufficient quantities of vaccine doses are not straight forward: at present the combined vaccine production capacity of all manufacturers is not sufficient to timely provide for a worldwide vaccination campaign. There is a clear need for alternative vaccine delivery systems and production technologies that could help to overcome this problem.

Since different antigenically distinct clades of H5N1 viruses have been identified recently, an ideal vaccine would also induce cross-protective immunity against these antigenic variants. Recently, conventional inactivated vaccine preparations have been evaluated, like whole inactivated virus (WIV) and split virion vaccines (Nicholson, K. G., A. E. Colegate, A. Podda, I. Stephenson, J. Wood, E. Ypma, and M. C. Zambon. 2001. Safety and antigenicity of non-adjuvanted and MF59adjuvanted influenza A/Duck/Singapore/97 (H5N3) vaccine: a randomised trial of two potential vaccines against H5N1 influenza. Lancet 357:1937-43). These have the drawback that such a vaccine would require significant additional product characterization with regard to possible shelf life. Furthermore, implementation of such new processes by vaccine producers is not necessarily successful and tests for validation would need to be redone. In light of the danger of a pandemic these drawbacks are significant. A safe and effective adjuvant could improve the immunogenicity of conventional vaccines and may reduce the antigen-quality required to induce adequate antibody responses (dose sparing). The results herein underscore this possibility (Stimune®-adjuvanted NIBRG-14 whole virus preparation). However, at present such potent adjuvanted formulations are not considered suitable for manufacturing and applications in humans and a vaccine that does not require an adjuvant for enhancement of its immunogenicity is a favourable candidate in future vaccine development.

Vaccines based on recombinant HA expressed by baculoviruses have been tested (Treanor, J. J., B. E. Wilkinson, F. Masseoud, J. Hu-Primmer, R. Battaglia, D. O'Brien, M. Wolff, G. Rabinovich, W. Blackwelder, and J. M. Katz. 2001. Safety and immunogenicity of a recombinant hemagglutinin vaccine for H5 influenza in humans. Vaccine 19:1732-7). In antigenically naïve individuals these vaccines were moderately immunogenic however, and more importantly appreciable antibody responses were only induced when high doses, or a combination with an adjuvant like alum was used (Nicholson, K. G., A. E. Colegate, A. Podda, I. Stephenson, J. Wood, E. Ypma, and M. C. Zambon. 2001. Safety and antigenicity of non-adjuvanted and MF59adjuvanted influenza A/Duck/Singapore/97 (H5N3) vaccine: a randomised trial of two potential vaccines against H5N1 influenza. Lancet 357:1937-43).

Clearly, novel vaccines are urgently needed to overcome a catastrophic shortage of vaccine in particular in the case of a H5N1 influenza pandemic. Also the drawbacks outlined above must be overcome quickly in order to avoid the consequences a pandemic would bring about.

### Description of the invention

The present invention relates to the field of therapeutics more in particular the field of vaccines, more in particular the field of influenza A vaccines, more in particular H5N1 vaccines.
Influenzavirus A is a genus of a family of viruses called Orthomyxoviridae in virus classification. Influenzavirus A has only one species in it; that species is called "Influenza A virus". Influenza A virus causes "avian influenza" (also known as bird flu, avian flu, Influenzavirus A flu, type A flu, or genus A flu). It is hosted by birds, but may infect several species of mammals. All known subtypes are endemic in birds.

The present invention solves the above outlined problems. It teaches the use of a recombinant modified vaccinia virus Ankara (MVA) comprising a heterologous nucleic acid for the production of a medicament in particular a vaccine, wherein the sequence of heterologous nucleic acid is from influenza A virus class H5 antigen.

Recombinant modified vaccinia virus Ankara (MVA) comprising a heterologous nucleic acid, wherein (a) the heterologous nucleic acid is incorporated into a non-essential site within the genome of MVA, (b) the heterologous nucleic acid is under the control of a vaccinia virus promoter, or orthopoxvirus promoter, or poxvirus-specific promoter and, (c) the heterologous nucleic acid is selected from the group of nucleic acids encoding a gene or a part of a gene from an influenza A virus class H5.

### Detailed description of the invention

The inventors have found that the use of a recombinant modified vaccinia virus Ankara (MVA) comprising a heterologous nucleic acid for the production of a medicament in particular a vaccine, wherein the sequence of heterologous nucleic acid is from influenza A virus class H5 antigen solves the problems outlined above. In particular vaccination with MVA producing the HA of influenza H5N1 viruses induced potent antibody responses, even after a single vaccination, which correlated with protection against homologous and heterologous challenge infection. The invention relates to the use of a recombinant modified vaccinia virus Ankara (MVA) comprising a heterologous nucleic acid for the production of a medicament in particular a vaccine, wherein the sequence of heterologous nucleic acid is from influenza A virus class H5 antigen, for the treatment in particular of the avian flu in humans but also other animals.

MVA has been originally tested in over 120,000 individuals as particularly safe vaccine against human smallpox (Mayr, A., and K. Danner. 1978. Vaccination against pox diseases under immunosuppressive conditions. Dev Biol Stand 41:225-34).

The advantages of using MVA vector vaccines include their established safety profile in humans, their efficacy upon delivery of heterologous antigens in clinical tests, and the availability of technologies for large scale vaccine production under the requirements of GMP.

The inventors have constructed different recombinant MVA viruses expressing also the HA gene sequences of H5N1 influenza viruses A/Hongkong/156/97 (A/HK/156/97) or A/Vietnam/1194/04 (A/VN/1194/04). These viruses served as candidate vaccines in a mouse model to assess the induction of protective immunity against three different H5N1 viruses. A two-dose immunization regimen induced strong antibody responses that partially cross-reacted with heterologous H5N1 viruses; the elicited antibody responses correlated with protection against challenge infection with homologous and heterologous influenza viruses.

The vaccine according to the invention (MVA) may induce a broad immune response that protects individuals from severe clinical signs and histopathological changes in the respiratory tract even when the strains causing the infections do not fully match the vaccine antigen. The recombinant MVA vectors according to the invention have a number of properties that make them favorable vaccine candidates for use in humans.

First, recombinant MVA according to the invention can be considered as extremely safe viral vectors because of their distinct replication deficiency in mammalian cells and their well established avirulence *in vivo* including the safety of MVA in immunesuppressed macaques (Stittelaar, K. J., T. Kuiken, R. L. de Swart, G. van Amerongen, H. W. Vos, H. G. Niesters, P. van Schalkwijk, T. van der Kwast, L. S. Wyatt, B. Moss, and A. D. Osterhaus. 2001. Safety of modified vaccinia virus Ankara (MVA) in immunesuppressed macaques. Vaccine 19:3700-9.) or the innocuous application of high doses of recombinant MVA to HIV-infected individuals (5, Harrer E et al 2005 Antivir Ther 10:285; Dorrell et al. 2006 J Virol 80:4705).

Second, industrial scale manufacturing of MVA vaccines appears very feasible in recognition of the efforts undertaken to develop MVA as third generation vaccine against orthopoxvirus-related biothreat (Vollmar J et al. 2006 Vaccine 2006 Mar 15;24(12):2065).

Third, MVA vector vaccines according to the invention can deliver multiple heterologous antigens and allow for simultaneous induction of high level humoral and cellular immunity providing the possibility to develop multivalent vaccines.

Moreover, the production of MVA-based vaccines is independent of existing production capacity for conventional influenza vaccines. This way the production of MVA-based vaccines can help to reduce the envisaged shortage of vaccine doses in the time of an emerging pandemic. Another advantage is that the excellent immunogenicity of these vaccines is independent of the use of adjuvants.

A modified vaccinia virus Ankara (MVA) is a chicken cell adapted strain of vaccinia virus. Because of its avirulence found upon inoculation of animals and its striking deficiency to produce substantial amounts of new viral progeny in most cells of mammalian origin, MVA can be used under laboratory conditions of biosafety level 1. MVA serves as an efficient vector virus for expression of recombinant genes (Zota and Moss, 1992) and as candidate recombinant vaccine (Moss et al., 1996) with high safety profiles since MVA has been tested for pre-immunization in over 100000 humans being vaccinated against smallpox without causing notable side-effects. Several MVA vector vaccines have already entered clinical evaluation (McConkey et al., 2003, Cosma et al., 2003). Most recently, MVA is reassessed as candidate second generation vaccine against smallpox. According to the invention, the heterologous nucleic acid according to the invention is incorporated into a non-essential region of the genome of the MVA. According to the present invention, any MVA strain may be used.

WO 03/097844 A1 discloses a number of MVA strains on p. 4. One strain that may be used according to the present invention is the MVA-BN strain or a derivative thereof (WO 02/42480). Non-essential regions according to the present invention may be selected from (i) natural occurring deletion sides of the MVA genome with respect to the genome of the vaccinia virus strain Copenhagen or (ii) intergenic regions of the MVA genome. The term "intergenic region" refers preferably to those parts of the viral genome located between two adjacent genes that comprise neither coding nor regulatory sequences. However, the insertion sides for the incorporation of the heterologous nucleic acid according to the invention (non-essential region) are not restricted to these preferred insertion sides since it's within the scope of the present invention that the integration may be anywhere in the viral genome as long as it is possible to obtain recombinants that can amplified and propagated in at least one cell culture system, such as Chicken Embryo Fibroblasts (CEF cells). Thus, a non-essential region may also be a non-essential gene or genes, the functions of which may be supplemented by the cell system used for propagation of MVA.

In a preferred embodiment the heterologous nucleic acid is incorporated into a non-essential site within the genome of MVA.

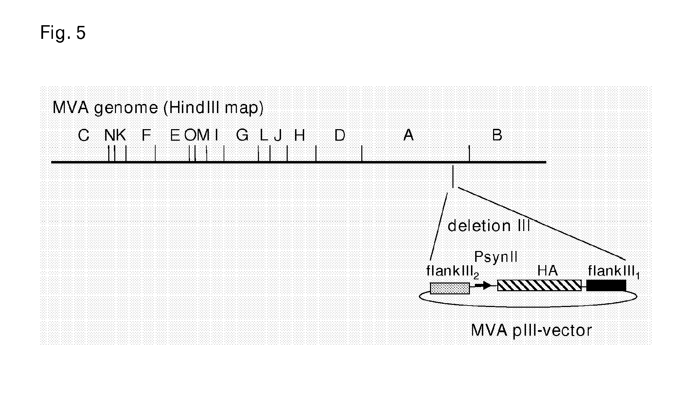

In a particularly preferred embodiment the heterologous nucleic acid according to the invention is incorporated into the MVA genome at the side of deletion (III). Integration of the heterologous nucleic acid according to the invention is performed preferentially by homologous recombination of the flanking regions of a so-called transfer vector, which initially comprises the heterologous nucleic acid according to the invention prior to its integration into the MVA genome. Upon homologous integration selection of recombinant viruses is performed. One such method for selecting the recombinant viruses is expression of the host range gene K1 L (see Fig. 5).

In a preferred embodiment the heterologous nucleic acid is incorporated into the MVA genome at the site of deletion III.

According to the invention the the heterologous nucleic acid is under the control of a vaccinia virus-, or orthopoxvirus-, or poxvirus-specific promoter. A number of promoters may be used for the present invention. For the expression of the heterologous nucleic acid according to the invention several promoters, such the 30K and 40K promoters (US 5,747,324, A Strong Synthetic Early-Late Promoter (Zota, et al., Vaccine (1994), 12, 1032-1040)), the P7.5 promoter (Endo et al., J. Gen. Virol. (1991) 72, 699-703) and a promoter derived from the cow pox virus type A (inclusion ATI gene) (Lee et al., J. Gen. Virol. 1998), 79, 613). All of these promoters may be used according to the invention. In one embodiment it is desired that the heterologous nucleic acid is expressed in high amounts. WO 03/097844 A1 discloses such a promoter.

According to the invention the sequence of the heterologous nucleic acid is selected from the group of H5N1, H5N3, H5N2, H5N7, H7N1,H7N7, H7N3 and H9N2 sequences. Preferably the sequence is from H5N1.

If the heterologous nucleic acid is from H5N1 it is preferably selected from the group of strains termed, A/Vietnam/1203/04, A/Vietnam/1194/04, A/Vietnam/3046/04, A/Hongkong/156/97, A/HongKong/212/03, A/HongKong/213/03, A/Indonesia/5/05 , A/Ck/Indonesia/BL03/03, A/Grey heron/HK/793.1/02, A/Black Headed
Gull/HK/12.1/03, A/Dk/Vietnam/11/04, A/Ck/Vietnam/33/04, A/Ck/Vietnam/C-58/04, A/Dk/TH/D4AT/04, A/Gs/TH/G7CS/04, A/Dk/FJ/1734/05, A/Ck/GX/463/06 and A/Ck/HK/947/06.

It is preferred that the heterologous nucleic acid of H5N1 is selected from strain A/Vietnam/1194/04, A/Indonesia/5/2005, A/Bar headed goose/Qinghai/1A/2005, A/Whooper swan/Mongolia/244/2005, A/turkey/Turkey/1/2005, and A/Anhui/1/2005.

It is most preferred that the heterologous nucleic acid of H5N1 is that of strain A/Vietnam/1194/04.

It is preferred that the sequence of the heterologous nucleic acid encompasses the sequence or part of the sequence of the hemagglutinin gene. In a preferred embodiment the sequence of the heterologous nucleic acid encompasses the sequence or part of the sequence of the hemagglutinin gene from nucleotide 1 to 1500. In a more preferred embodiment from nucleotide 1 to 1100 and in a most preferred embodiment from nucleotide 40 to 1100. In a most preferred embodiment the heterologous nucleic acid encompasses the sequence or part of the sequence of the hemagglutinin gene from A/Vietnam/1194/04 selected from SEQ ID NO. 1, SEQ ID NO. 2 and SEQ ID NO. 3. SEQ ID NO. 3 is most preferred (see table 1).

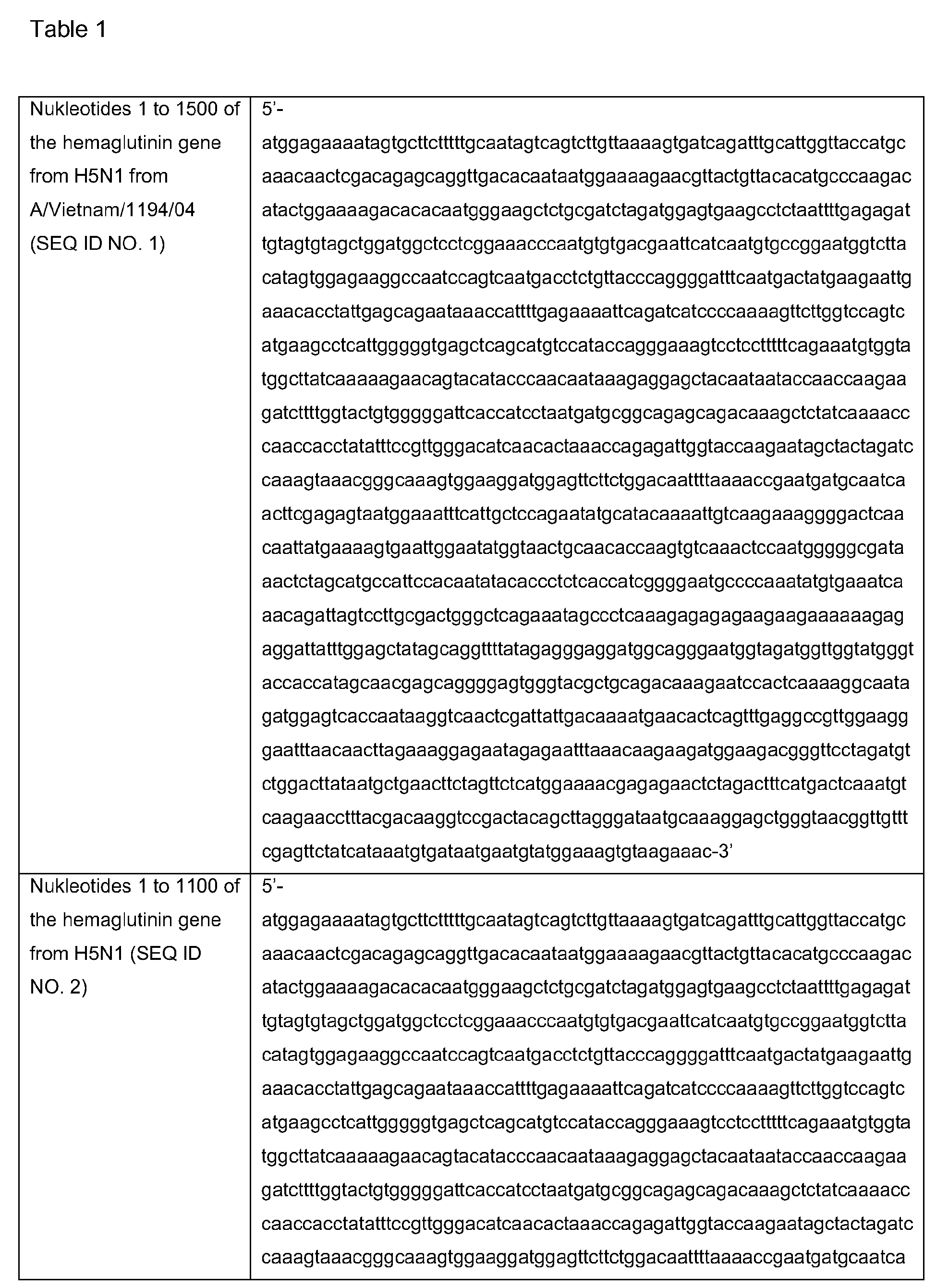

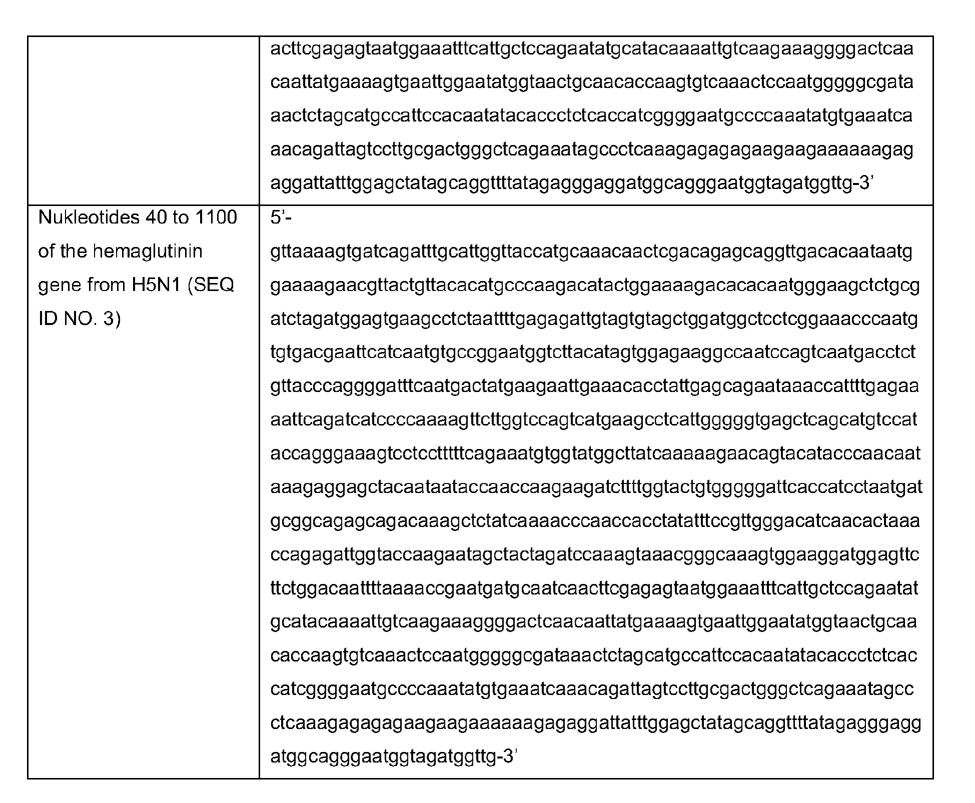

Hemagglutinin (HA) or haemagglutinin (BE) is an antigenic glycoprotein found on the surface of the influenza viruses (as well as many other bacteria and viruses). It is responsible for binding the virus to the cell that is being infected. The name hemagglutinin comes from the protein's ability to cause erythrocytes to clump together (Nelson DL and Cox MM, 2005. Lehninger's Principles of Biochemistry, 4th edition, WH Freeman, New York, NY.).

There are at least 16 different HA antigens. These subtypes are labeled H1 through H16. The last, H16, was discovered only recently on influenza A viruses isolated from black-headed gulls from Sweden and Norway (Fouchier RAM, Munster V, Wallensten A, et al, 2005. Characterization of a novel influenza A virus hemagglutinin subtype (H16) obtained from black-headed gulls. J Virol vol 79, issue 5, pp2814-22.). The first three hemagglutinins, H1, H2, and H3, are found in human influenza viruses.

A single amino acid change in the avian virus strain type H5 hemagglutinin has been found in human patients that can significantly alter receptor specificity of avian H5N1 viruses, providing them with an ability to bind to receptors optimal for human influenza viruses (Gambaryan A, Tuzikov A, Pazynina G, Bovin N, Balish A, Klimov A, 2005. Evolution of the receptor binding phenotype of influenza A (H5) viruses in Virology). This finding seems to explain how an H5N1 virus that normally does not infect humans can mutate and become able to efficiently infect human cells. The hemagglutinin of the H5N1 virus has been associated with the high pathogenicity of this flu virus strain, apparently due to its ease of conversion to an active form by proteolysis (Hatta M, Gao P, Halfmann P, Kawaoka Y, 2001. Molecular Basis for High Virulence of Hong Kong H5N1 Influenza A Viruses in Science vol 293, pp1840-1842).

It is preferred that the nucleic acid encompasses HA1 and HA2 subunits. HA is a homotrimeric integral membrane glycoprotein. It is shaped like a cylinder, and is approximately 135 A (angstroms) long. The three identical monomers that constitute HA are constructed into a central α helix coil; three spherical heads contain the sialic acid binding sites. HA monomers are synthesized as precursors that are then glycosylated and cleaved into two smaller polypeptides: the HA1 and HA2 subunits. Each HA monomer consists of a long, helical chain anchored in the membrane by HA2 and topped by a large HA1 globule. It is also preferred that the HA1 subunit is used alone.

In further embodiment the heterologous nucleic acid encompasses the sequence coding for an amino acid sequence region selected from amino acids 1-351, amino acids 352-500, amino acids 15-351, amino acids 120-160, amino acids 180-230 and amino acids 120-230. The following are most preferred amino acids 120-160, amino acids 180-230 and amino acids 120-230. The very most preferred region is 120-160. It is most preferred that the heterologous nucleic acid encodes an amino acid sequence selected from SEQ ID NO. 4, SEQ ID NO. 5, SEQ ID NO. 6, SEQ ID NO. 7 and SEQ ID NO. 8.

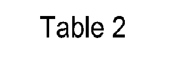

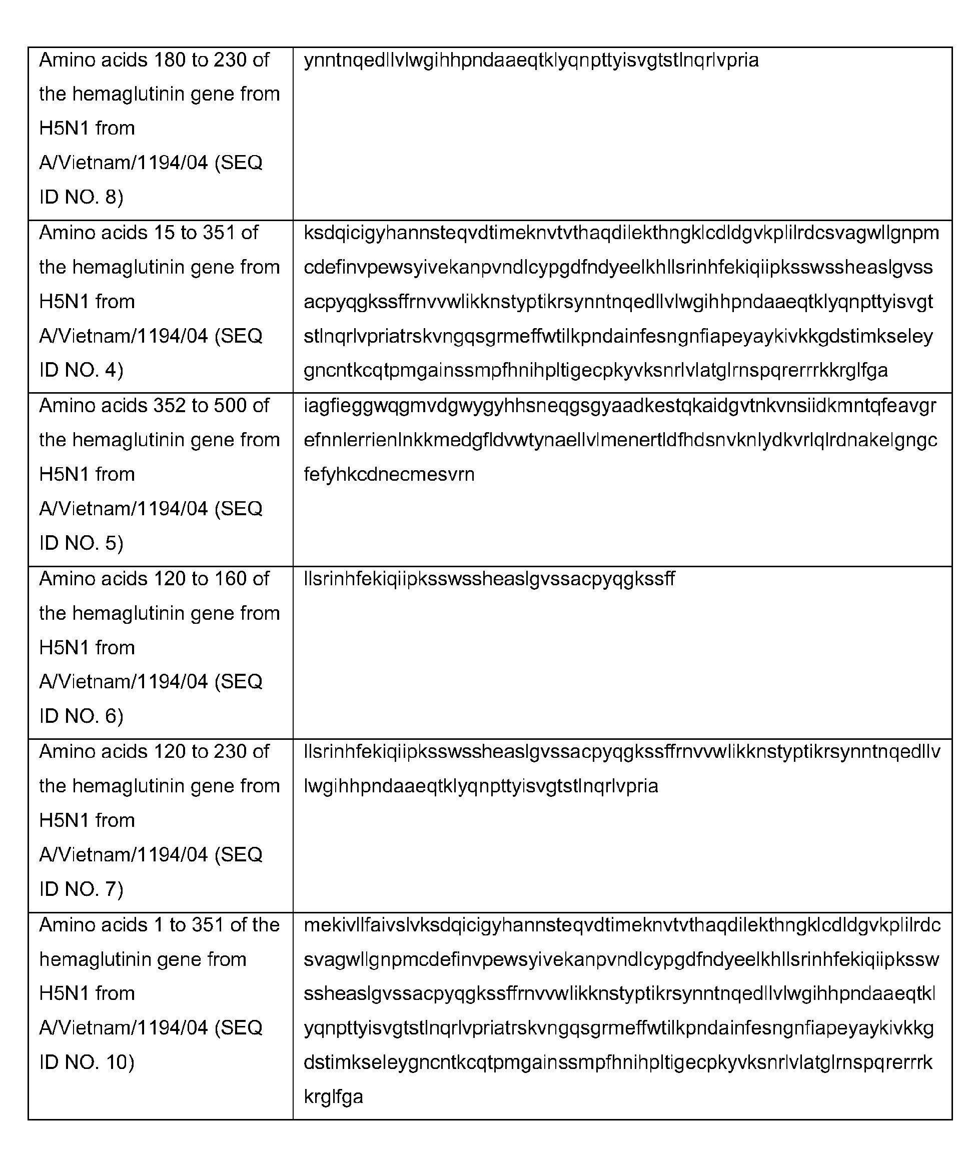

In particularly preferred embodiment the heterologous nucleic acid encodes two or more sequences stemming from (a) different genes or parts thereof of a single H5N1 strain and/or, (b) different H5N1 strains. It is particularly preferred to us two or more, three or more or four or more HA sequences from different H5N1 strains. Further the two or more different sequences may be from HA and another gene of the virus.

In a preferred embodiment the sequence of the heterologous nucleic acid encompasses the sequence or part of the sequence of the hemagglutinin gene from nucleotide 1 to 1500. In a more preferred embodiment from nucleotide 1 to 1100 and in a most preferred embodiment from nucleotide 40 to 1100.

A preferred sequence is the neuraminidase (NA). NA codes for neuraminidase which is an antigenic glycoprotein enzyme found on the surface of the influenza viruses. It helps the release of progeny viruses from infected cells. NA is considered as possible target antigen to elicit influenza virus-specific immune responses, i.e. antibodies and T cells, that may contribute to cross-protection against different virus variants or subtypes.

Preferred sequences are matrix sequences. Matrix encoding gene segments are matrix proteins (M1 and M2) that along with the two surface proteins (hemagglutinin and neuraminidase) make up the capsid (protective coat) of the virus. M1 is a protein that binds to the viral RNA.
M2 is a protein that uncoats the virus exposing its contents (the eight RNA segments) to the cytoplasm of the host cell. The M2 transmembrane protein is an ion channel required for efficient infection (Influenza virus replication in Medical Microbiology, 4th edition edited by Samuel Baron. 1996 Chapter 58. ISBN 0-9631172-1-1). The amino acid substitution (Ser31Asn) in M2 some H5N1 genotypes is associated with amantadine resistance (H5N1 influenza: A protean pandemic threat National Academy of Sciences, PNAS, May 25, 2004, vol. 101, no. 21, 8156-8161; Characterization of Highly Pathogenic H5N1 Avian Influenza A Viruses Isolated from South Korea Journal of Virology, March 2005, p. 3692-3702, Vol. 79, No. 6. Also, Pandemic Influenza Center for Infectious Disease Research & Policy Academic Health Center -- University of Minnesota). M1 and M2 are considered as possible target antigens to elicit influenza virus-specific immune responses, i.e. antibodies and T cells, that may contribute to cross-protection against different influenza virus variants or subtypes.

Thus in one embodiment two or more HA antigens are used. In one embodiment One or more HA antigens is combined with NA, M1, or M2 .

In a particularly preferred embodiment the two or more sequences encode hemagglutinin or a part of hemagglutinin and stem from, A/Vietnam/1203/04, A/Vietnam/1194/04, Vietnam/3046/04, A/Bar headed goose/Qinghai/1A/2005, A/Whooper swan/Mongolia/244/2005, A/turkey/Turkey/1/2005, A/Anhui/1/2005, and/or A/IND/5/05.

In a further aspect of the invention the invention relates to a recombinant modified vaccinia virus Ankara (MVA) comprising a heterologous nucleic acid, wherein (a) the heterologous nucleic acid is incorporated into a non-essential site within the genome of MVA, (b) the heterologous nucleic acid is under the control of a vaccinia virus promoter, or orthopoxvirus promoter, or poxvirus-specific promoter and, (c) the heterologous nucleic acid is selected from the group of nucleic acids encoding a gene or a part of a gene from an influenza A virus class H5. A modified vaccinia virus Ankara (MVA) is a chicken cell adapted strain of vaccinia virus. Because of its avirulence found upon inoculation of animals and its striking deficiency to produce substantial amounts of new viral progeny in most cells of mammalian origin, MVA can be used under laboratory conditions of biosafety level 1. MVA serves as an efficient vector virus for expression of recombinant genes (Sutter and Moss, 1992) and as candidate recombinant vaccine (Moss et al., 1996) with high safety profiles since MVA has been tested for pre-immunization in over 100000 humans being vaccinated against smallpox without causing notable side-effects. Several MVA vector vaccines have already entered clinical evaluation (McConkey et al., 2003, Cosma et al., 2003). Most recently, MVA is reassessed as candidate second generation vaccine against smallpox. According to the invention, the heterologous nucleic acid according to the invention is incorporated into a non-essential region of the genome of the MVA. According to the present invention, any MVA strain may be used.

WO 03/097844 A1 discloses a number of MVA strains on p. 4. One strain that may be used according to the present invention is the MVA-BN strain or a derivative thereof (WO 02/42480). Non-essential regions according to the present invention may be selected from (i) natural occurring deletion sides of the MVA genome with respect to the genome of the vaccinia virus strain Copenhagen or (ii) intergenic regions of the MVA genome. The term "intergenic region" refers preferably to those parts of the viral genome located between two adjacent genes that comprise neither coding nor regulatory sequences. However, the insertion sides for the incorporation of the heterologous nucleic acid according to the invention (non-essential region) are not restricted to these preferred insertion sides since it's within the scope of the present invention that the integration may be anywhere in the viral genome as long as it is possible to obtain recombinants that can amplified and propagated in at least one cell culture system, such as Chicken Embryo Fibroplasts (CEF cells). Thus, a non-essential region may also be a non-essential gene or genes, the functions of which may be supplemented by the cell system used for propagation of MVA.

It is preferred that the heterologous nucleic acid is selected from the group of H5N1, H5N3, H5N2, H7N7, H3N2 and H7N3 genome sequences.

It is preferred that the heterologous nucleic acid is from H5N1 and is selected from the group of strains termed, A/Vietnam/1203/04, A/Vietnam/1194/04, A/Vietnam/3046/04, A/Hongkong/156/97, A/HongKong/212/03, A/HongKong/213/03, A/Indonesia/5/05 , A/Ck/Indonesia/BL03/03, A/Grey heron/HK/793.1/02, A/Black Headed Gull/HK/12.1/03, A/Dk/Vietnam/11/04, A/Ck/Vietnam/33/04, A/Ck/Vietnam/C-58/04, A/Dk/TH/D4AT/04, A/Gs/TH/G7CS/04, A/Dk/FJ/1734/05, A/Ck/GX/463/06, A/Ck/HK/947/06.
In a further embodiment the heterologous nucleic acid is from H5N1 and is selected from the group of strains termed A/Vietnam/1194/04, A/Indonesia/5/2005, A/Bar headed goose/Qinghai/1A/2005, A/Whooper swan/Mongolia/244/2005, A/turkey/Turkey/1/2005, and A/Anhui/1/2005.

It is most preferred that the heterologous nucleic acid of H5N1 is that of strain A/Vietnam/1194/04.

In one embodiment the sequence of the heterologous nucleic acid encompasses the sequence or part of the sequence of the hemagglutinin gene (see above). In further embodiment the heterologous nucleic acid encompasses the sequence coding for an amino acid sequence region selected from amino acids 1-351, amino acids 352-500, amino acids 15-351, amino acids 120-160, amino acids 180-230 and amino acids 120-230. The following are most preferred amino acids 120-160, amino acids 180-230 and amino acids 120-230. The very most preferred region is 120-160.

Furthermore, a third H5N1variant strain was used for challenge infection of the mice: A/IND/5/05, which was antigenically distinct from the other two viruses. The recombinant MVA-HA-HK/97 was highly immunogenic. A single immunization already induced potent antibody responses against influenza virus A/HK/156/97, which were further boosted by a second vaccination. These antibodies were not cross-reactive in HI and VN assays with A/VN/1194/04 or A/IND/5/05. MVA-HA-VN/04 was less immunogenic, but after two immunizations substantial antibody responses were observed, not only against the homologous virus but also to A/HK/156/97 and to a lesser extent to A/IND/5/05. The observed antibody reactivity pattern is similar to that observed with post infection ferret sera. Thus, this asymmetry in antibody recognition pattern observed with antibodies induced by MVA-HA vaccination fully resembled that observed with antibodies induced after infection with the original influenza viruses.
The whole virus antigen NIBRG-14 vaccine preparation was included in the experiments as a positive control and was highly immunogenic in combination with the Stimune® water-in-oil adjuvant, also known as Specol being used in research for production of antigen-specific antibodies in laboratory animals. This experimental vaccine not only induced strong antibody responses to the homologous influenza virus A/VN/1194/04 but also to the other two H5N1 strains. The HI and VN antibody titers measured against the three H5N1 strains directly correlated with protection against challenge infection.
The MVA-HA-HK/97 immunized mice were only protected against a homologous challenge infection. Vaccination prevented virus replication completely and as a result neither histopathological changes nor clinical signs were observed in these mice. Although the MVA-HA-HK/97 induced antibodies did not cross-react with influenza virus A/VN/1194/04, replication of this virus was reduced and the immunized animals were protected from clinical signs (Table 1).

In contrast, no protective effects were seen against challenge infection with influenza virus A/IND/5/05. Although it is known that MVA vaccination can induce strong CTL responses which could have contributed to protection (3), it is unknown at present whether H-2b restricted crossreactive CTL epitopes exist on the HA molecule of influenza H5N1 viruses. Immunization with MVA-HA-VN/04 induced sterilizing immunity against the homologous strain. In addition, strong protective effects were observed against the antigenically distinct influenza viruses A/HK/156/97 and A/IND/5/05. The replication of these viruses was largely reduced in most immunized animals, which correlated with the absence of infected cells in the respiratory tract and the lack of clinical signs. The protection is most likely based on virus neutralizing HA-specific serum antibodies that transsudate from the circulation into the alveolar epithelium (Ito, R., Y. A. Ozaki, T. Yoshikawa, H. Hasegawa, Y. Sato, Y. Suzuki, R. Inoue, T. Morishima, N. Kondo, T. Sata, T. Kurata, and S. Tamura. 2003. Roles of antihemagglutinin IgA and IgG antibodies in different sites of the respiratory tract of vaccinated mice in preventing lethal influenza pneumonia. Vaccine 21:2362-71).

The HA sequences as well as the other sequences need not be present in their entirety. They may be for example codon optimized in order to be, e.g. better expressed.

It is preferred for the recombinant modified vaccinia virus Ankara (MVA) according to the invention the the nucleic acid encompasses HA1 and HA2 subunits.

In one embodiment the nucleic acid of the recombinant modified vaccinia virus Ankara (MVA) according to the invention, encodes two or more sequences stemming from (a) different genes or parts thereof of a single H5N1 strain and/or, (b) different H5N1 strains. Such embodiments have been outlined in detail above.

In a preferred embodiment the two or more sequences encode hemagglutinin or a part of hemagglutinin and stem from, A/Vietnam/1203/04, A/Vietnam/1194/04, A/Vietnam/3046/04, A/Hongkong/156/97, A/HongKong/212/03, A/HongKong/213/03, A/Indonesia/5/05 , A/Ck/Indonesia/BL03/03, A/Grey heron/HK/793.1/02, A/Black Headed Gull/HK/12.1/03, A/Dk/Vietnam/11/04, A/Ck/Vietnam/33/04, A/Ck/Vietnam/C-58/04, A/Dk/TH/D4AT/04, A/Gs/TH/G7CS/04, A/Dk/FJ/1734/05, A/Ck/GX/463/06 or A/Ck/H K/947/06.

In one embodiment the invention relates to a nucleic acid encoding a modified vaccinia virus Ankara (MVA) according to the invention outlined above.

In a further embodiment the invention relates to a method of introducing the recombinant MVA according to the invention into a target cell comprising infection of the target cell with a recombinant MVA according to the invention.

The invention also relates to method for immunization of a living animal body including a human said method comprising administering to said living animal body including a human in need thereof a therapeutically effective amount of the MVA according to the invention.

In a further embodiment the invention relates to a method of introducing a MVA according to the invention into a target cell comprising infection of the target cell with an MVA according to the invention.
Viruses are propagated in titered following standard methodology. To generate vaccine preparations, viruses may routinely be purified by ultracentrifugation through sucrose and reconstituted in, e.g. 1 mmol tris pH 9,0. CEN rabid kidney RK-13 (Atcc ccl-37) cells may be grown in minimal essential media (MEM) supplemented with 10 % fibroplast serum (FCS) and maintained at 37°C and 5 % CO₂. CEF cells may be grown in 6 well tissue culture plates and infected with the multiplicity of 20 infectious units MVA.

The determined low or high multiplicity growth profiles, confluent CEF monolayers (grown on 6 well plates) may be infected with 0,05 infectious units (IU) or 10 IU MVA or MVA according to the invention per cell, respectively. After virus absorption for 60 min at 37°C the inoculum may be removed. Cells may be washed twice with RPME 6040 and incubated with fresh RPME 6040 medium containing 10 % FCS at 37°C and 5 % CO₂. At multiple time points post infection (p.i.) infected cells may be harvested and virus may be released by freeze-drying and a brief sonication. Serial dilutions of the resulting lysates may be plated on confluent CEF monolayers grown in 6 well plates as replicates of two 48 hrs p.i. monolayers may be briefly fixed in acetone: methanol (1:1) and cells may be incubated for 60 min with bodyclonal rabbit anti-vaccinia antibody (IgG fraction, Biogenesis Ltd., Pool, England), followed by an incubation for 45 min horseradish-peroxidase-conjugated polyclonal goat anti-rabbit antibody (Dianova, Hamburg). After washing with PBS, antibody-labeled cells may be developed using an o-dianisidine (Sigmar, Taufkirchen, Germany) substrate solution, foci of stained cells may be counted, and virus titers may be calculated as IU/ml.

According to one embodiment of the present invention, the invention relates to a method for immunization of a living animal body including a human, said method comprising administering to said living animal body including a human in need of a therapeutically effective amount of the MVA according to the invention. Said method according to the invention may comprise a composition which may also contain (in addition to the ingredient and the carrier) diluents, common fillers, salts, buffers, stabilizers, solubilizers and other materials well known in the art. It is necessary that these are pharmaceutically acceptable. The term "pharmaceutically acceptable" means a non-toxic material that does not interfere with the effectiveness of the biological activity of the MVA according to the invention. The characteristics of the carrier will depend on the route of administration. The pharmaceutical composition may further contain other agents which either enhance the activity or use in treatment. Such additional factors and/or agents may be included in the pharmaceutical composition to be applied for the method for immunization according to the invention to produce a synergistic effect or to minimize side-effects. Techniques for formulation and administration of the MVA according to the invention may be found in "Remington's Pharmaceutical Sciences", (Muck Publishing Company, Easton, PA, latest edition).

The method for immunization according to the present invention will make use of a therapeutically effective amount of the MVA. A therapeutically effective dose further refers to that amount of the compound/ingredient sufficient to result in amelioration of symptoms, e.g. treatment, healing, prevention or amelioration of such conditions. To prepare vaccines, the MVA vaccinia virus generated according to the invention are converted into a physiologically acceptable form. This can be done based on the many years of experience in the preparation of vaccines used for vaccination against smallpox (Kaplan, B. R. Med. Bul. 25, 131-135 [1996]). Typically, about 10⁶ to 10⁷ particles of the recombinant MVA are freeze-dried in 100 ml of phosphate-buffered saline (PBS) in the presence of 2 % peptone and 1 % human albumin in an ampoule, preferably a glass ampoule The lyophilisate can contain extenders, such as manitol, dextran, sugar, glycine, lactose or polydinylpyrolidole or other aids (such as antioxidants, stabilizers, etc.) suitable for parenteral administration. The glass ampoule is then sealed and can be stored, preferably at temperatures below -20°C for several months. For vaccination the lyophilisate can be dissolved in 0,1 to 0,2 ml of aqueous solution, preferably physiological saline, and administered parentally, for example by intradermal inoculation. The vaccine according to the invention is preferably injected intracutaneously. Slight swelling and redness, sometimes also itching, may be found at the injection side. The mode of administration, the dose and the number of administrations can be optimized by those skilled in the art in a known manner. It is expedient where appropriate to administer the vaccine several times over a lengthy period in order to obtain a high-level immune response against the foreign antigen. In a preferred embodiment the immunizing may be both prophylactic and/or therapeutic.

The invention relates also to a vaccination kit with the vaccine, pre-filled syringes with the vaccine and other modes of storage and application.

In one embodiment the living animal body is a mammal, in one it is a bird. In a preferred embodiment it is a chicken or a human.

In one embodiment said immunizing may be both prophylactic and/or therapeutic.

### Examples

Vaccine preparation

The H5N1 viruses were propagated in MDCK cells and the viral RNA was extracted from the culture supernatants using a high pure RNA isolation kit (Roche, Almere, the Netherlands). Subsequently cDNA was synthesized from the vRNA using Superscript reverse transcriptase (Invitrogen, Carlsbad, California, USA) and the 5'-AGCAAAAGCAGG-3' (SEQ ID NO. 9) oligonucleotide (Eurogentec, Seraing, Belgium) as primer. Next, the HA gene sequences were amplified by polymerase chain reaction using Pfu (Stratagene, La Jolla, California, USA) as heat stable DNA polymerase. Primer sequences were extended with the Notl and Xhol restriction sites to facilitate directional cloning into the cloning plasmid pBluescriptSK+ (Stratagene, La Jolla, California, USA).

HA gene sequences were prepared from these plasmids by Notl /Xhol digestion, treated with Klenow polymerase to generate blunt ends and cloned into the Pmel site of MVA expression plasmid pIIIdHR-Psynll to generate the MVA vector plasmids plll-HA-HK/97 and plll-HA-VN/04.

Upon transfection in MVA-infected cells these plasmids direct insertion of foreign genes into the site of deletion III within the MVA genome (Sutter, G., and B. Moss. 1992. Nonreplicating vaccinia vector efficiently expresses recombinant genes. Proc Natl Acad Sci U S A 89:10847-51.) and allow transcription of the HA target genes under control of the vaccinia virus-specific promoter Psynll (Wyatt et al. 2004, AIDS Res Hum Retr 20, 645-653). Recombinant viruses MVA-HA-HK/97and MVA-HA-VN/04 were generated in primary chicken embryo fibroblasts (CEF) upon transfection with 1 yg plasmid DNA, infection with 0.05 infectious units / cell MVA isolate F6 (Sutter, G., and B. Moss. 1992. Nonreplicating vaccinia vector efficiently expresses recombinant genes. Proc Natl Acad Sci U S A 89:10847-51.), and by plaque selection on RK-13 cells (Staib, C., I. Drexler, and G. Sutter. 2004. Construction and isolation of recombinant MVA. Methods Mol Biol 269:77-100). The recombinant MVA genomes were analyzed by PCR to verify HA gene insertion and genetic stability (data not shown). The production of HA antigens by the MVA vector viruses was confirmed by Western blot analysis of CEF cell lysates harvested at various time points after infection with MVA-HA-HK/97 or MVA-HA-VN/04 (data not shown). One-step and multiple-step growth analysis in CEF demonstrated close to identical replicative capacities of MVA-HA-HK/97and MVA-HA-VN/04 in comparison to non-recombinant MVA (data not shown).

To generate vaccine preparations the viruses were amplified in CEF, purified by ultracentrifugation through sucrose and reconstituted in 1 mM Tris/HCI pH 9.0. MVA vaccines were used at a dose of 10⁸ PFU diluted in 100µl PBS.

Whole inactivated NIBRG-14 virus, a reassortant vaccine strain based on influenza virus A/Vietnam/1194/04 made by reverse genetics was used as positive control. The lyophilised whole virus antigen was reconstituted in distilled water at a concentration of 2µg WAS THIS HA?/50µl and mixed 1:1 with the adjuvant: Stimune® (Cedi-Diagnostics, Lelystad, The Netherlands). Control mice were inoculated with PBS.

### Influenza viruses

Influenza viruses A/HK/156/97, A/VN/1194/04 and A/Indonesia/5/05 (A/IND/5/05) were inoculated in the allantoic cavity of 11-day-old embryonated chicken eggs. The allantoic fluid was harvested after 3 days. Infectious virus titers were determined in Madin-Darby Canine Kidney (MDCK) cells as described previously (Rimmelzwaan, G. F., M. Baars, E. C. Claas, and A. D. Osterhaus. 1998. Comparison of RNA hybridization, hemagglutination assay, titration of infectious virus and immunofluorescence as methods for monitoring influenza virus replication in vitro. J Virol Methods 4:57-66).

Mice

Female specified pathogen free 6-8 weeks old C57BL/6J mice were purchased from Charles River (Sulzfeld, Germany). The animals (n=90) were divided in five groups of 18 mice and immunized with either PBS, MVA-HA-HK/97, MVA-HA-VN/04, wtMVA, or Stimune®-adjuvanted NIBRG-14.
Immunization was done intramuscular, 50µl in the left hind leg and 50µl in the right. Four weeks later, blood was drawn by orbita puncture and animals were immunized again as described above. Another four weeks later blood was drawn and each of the five vaccine groups (n=18) was divided into three sub-groups of six animals each.
The sub-groups of each vaccine group were inoculated with 10³ TCID50 of influenza virus A/HK/156/97, A/VN/1194/04 or A/IND/5/05 in 50µl PBS by the intranasal route. Six nonimmunized animals were used as negative controls and were inoculated with 50µl PBS. Animals were weighed every day until day 4 after infection and then sacrificed by exsanguination.
After sacrificing the mice the following organs were resected: brain, lungs (inflated with formalin), spleen and intestines. Animals in all groups were properly age-matched at the time point of challenge infection.
The experimental protocol was approved by an independent Animal ethics committee prior to the start of the experiment. Intramuscular immunizations, intranasal infections, orbita punctures and euthanasia were carried out under anaesthesia with inhalative isoflurane. The animals were housed in filter-top cages and had access to food and water *ad libitum.* During the 5 days of infection with the H5N1 influenza virus, animals were placed in filter-top cages in biosafety level 3 containment facilities. One BSL-3 isolator unit was used per virus.

### Virus titers in organ tissues

Organs were snap frozen on dry ice with ethanol and stored at -70°C. Organs were homogenized with a Polytron homogenizer (Kinematica AG, Littau-Lucerne, Switzerland) in transport medium (Hanks medium (MEM), Glycerol, 100U/ml penicillin, 100µg/ml streptomycin, polymyxin B, nystatin, gentamicin, 7,5% NaHC03, 1M Hepes). Quintuplicate ten-fold serial dilutions of these samples were used to determine the virus titers on confluent layers of Madin-Darby Canine Kidney Cells (MDCK cells).

### Serology

After treatment with cholera filtrate and heat-inactivation at 56°C, the sera were tested for the presence of anti-HA antibodies. For this purpose a hemagglutination inhibition assay (HI) was used following a standard protocol using 1% turkey erythrocytes and four HA-units of either influenza virus A/HK/156/97, A/VN/1194/04 or A/IND/5/05 (Palmer, D., et al. 1975. Haemagglutination inhibition test, p. 25-62, Advanced laboratory techniques for influenza diagnosis. Procedural Guide. US Dept. Hlth. ED., Atlanta). For this purpose reverse genetics viruses were produced from which the basic cleavage site was removed. The use of these reverse genetics viruses was validated and titers obtained were comparable with those against the wildtype strains (data not shown). Sera were also tested for the presence of virus neutralising antibodies specific for the three influenza viruses using a micro virus neutralisation (VN) assay with 100 TCID₅₀ of the respective viruses (Frank, A. L., J. Puck, B. J. Hughes, and T. R. Cate. 1980. Microneutralization test for influenza A and B and parainfluenza 1 and 2 viruses that uses continuous cell lines and fresh serum enhancement. J Clin Microbiol 12:426-32). Hyperimmune serum obtained from a swan immunized twice with inactivated H5N2 influenza virus A/Duck/Potsdam/1402/86 (Intervet, Boxmeer, the Netherlands) was used as a positive control against the 3 different influenza A viruses.

### Histopathology

Formalin-inflated lungs were fixated in 10% neutral buffered formalin, embedded in paraffin, sectioned at 4µm and stained with hematoxylin and eosin for histological evaluation. Furthermore slides were stained using an immunoperoxidase method with a monoclonal antibody (Clone HB65 IgG2a (American Type Culture Collection)) directed against the nucleoprotein of influenza A virus. A Goat-anti-mouse IgG2a HRP (Southern Biotech, Birmingham, Alabama, USA) was used as the secondary antibody. The peroxidase was revealed using diamino-benzidine as a substrate, resulting in a deep red precipitate in the nuclei of influenza A infected cells and a less intense red staining of the cytoplasm. The sections were counterstained with hematoxylin.

### Statistical analysis

Data for viral titers and antibody titers were analysed using the two-sided Student's *t* test and differences were considered significant at P < 0.05.

### Clinical signs

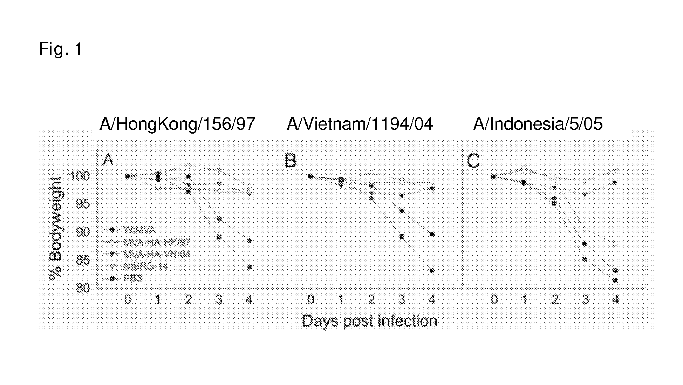

From day two post infection onwards, mice immunized with PBS or wtMVA developed clinical signs like hunched posture, rapid breathing, ruffled fur and decreased muscle strength irrespective of the influenza H5N1 virus that was used for infection. These clinical signs were not observed in mice infected with influenza virus A/HK/156/97 or A/VN/1194/04 after vaccination with MVA-HA-HK/97 or MVA-HA-VN/04. MVA-HA-VN/04 vaccination also prevented the development of clinical signs caused by infection with influenza virus A/IND/5/05. The observed protection against clinical signs correlated with reduced loss of bodyweight after infection (Figure 1). In PBS and wtMVA immunized mice an average loss of bodyweight of 16.2% and 11.5% was observed post infection with influenza virus A/HK/156/97 (Figure 1A) or 16.9% and 10.4% post infection with influenza virus A/VN/1194/04 (Figure 1B), respectively. This loss of bodyweight was significantly reduced by vaccination with MVA-HA-HK/97 or MVA-HA-VN/04 (Figure 1). Also infection with influenza virus A/IND/5/05 (Figure 1C) caused severe loss of bodyweight in PBS-immunized control mice or wtMVA vaccinated mice (16.9% and 18.6% respectively), which was significantly reduced by vaccination with MVA-HA-VN/04 but not by vaccination wit MVA-HA-HK/97.

### Virus replication in organs

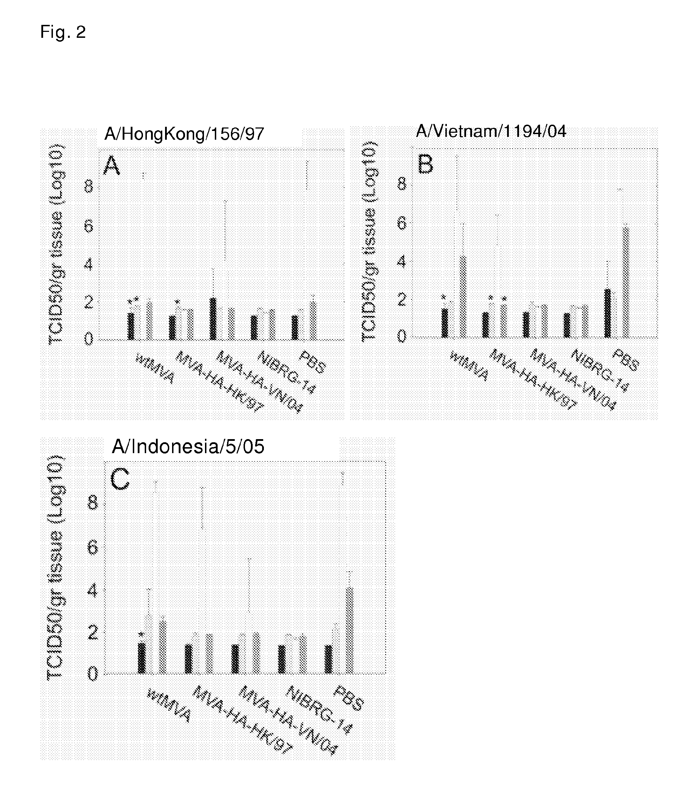

Infectious virus titers were determined in brains, intestines, lungs and spleens on day 4 post infection with influenza viruses A/HK/156/97 (Figure 2A), A/VN/1194/04 (Figure 2B) or A/IND/5/05 (Figure 2C).
After infection, the highest virus replication was observed in the lungs with average lung virus titers of 10^{7.9}, 10^{7.8} and 10^{8.9} TCID50/gram tissue for unprotected PBS-inoculated mice subsequently infected with influenza viruses A/HK/156/97, A/VN/1194/04 or A/IND/5/05 respectively. Also mice vaccinated with wtMVA were not protected and similar average virus titers were found in the lungs of infected mice. In some animals in both groups of unprotected mice virus replication could be demonstrated in extra-respiratory tissues including brain, intestines and spleen (Table 1).

**Table 1**

| | | | | | | | | | | | | | | | |
|---|---|---|---|---|---|---|---|---|---|---|---|---|---|---|---|
| Table 1 shows detection of infectious virus and weight loss observed in individual mice. Group numbers < 6 are caused by fatalities due to complications that had nothing to do with the experiment. | | | | | | | | | | | | | | | |

| Virus used for challenge infection | | | | | | | | | | | | | | | |
|---|---|---|---|---|---|---|---|---|---|---|---|---|---|---|---|
| | A/HK/156/97 | | | | | A/VN/1194/04 | | | | | A/IND/5/05 | | | | |
| Vaccine group | brain loss | intestines | lung | spleen | >10% weight | brain loss | intestines | lung | spleen | >10% weight | brain loss | intestines | lung | spleen | 10% weight |
| wtMVA | 1/6 | 1/6 | 6/6 | 4/6 | 5/6 | 2/6 | 0/6 | 6/6 | 3/6 | 4/6 | 2/6 | 2/6 | 6/6 | 4/6 | 5/6 |
| MVA-HA- HK/97 | 0/4 | 0/4 | 0/4 | 0/4 | 0/4 | 0/6 | 1/6 | 6/6 | 1/6 | 1/6 | 0/6 | 0/6 | 6/6 | 0/6 | 4/6 |
| MVA-HA- VN/04 | 2/6 | 0/6 | 2/6 | 0/6 | 0/6 | 0/6 | 0/6 | 0/6 | 0/6 | 0/6 | 0/6 | 0/6 | 2/6 | 0/6 | 0/6 |
| NIBRG-14 | 0/6 | 0/6 | 0/5 | 0/6 | 0/6 | 0/6 | 0/6 | 0/6 | 0/6 | 0/6 | 0/6 | 0/6 | 0/6 | 0/6 | 0/6 |
| PBS | 0/6 | 0/6 | 5/5 | 3/6 | 4/5 | 3/5 | 2/5 | 5/5 | 3/6 | 5/5 | 0/6 | 3/6 | 6/6 | 4/6 | 6/6 |

Vaccination with MVA-HA-HK/97 prevented replication of influenza virus A/HK/156/97 in the lungs and other organs completely whereas with MVA-HA-VN/04 vaccination a reduction of virus replication in the lungs was observed in four out of six mice.
After challenge infection with influenza virus A/VN/1194/04 it was the other way around: vaccination with MVA-HA-VN/04 prevented replication completely, whereas vaccination with MVA-HA-HK/97 only partially reduced virus replication.
The reduction was statistically significant compared to PBS-inoculated mice (p < 0.05), while such a difference was not observed for wtMVA vaccinated mice.
Vaccination with MVA-HA-VN/04 also prevented replication of influenza virus A/IND/5/05 in the lungs of four out of six mice resulting in reduced average lungs titers compared to PBS and wtMVA vaccinated mice. Vaccination with MVA-HA-HK/97 did not prevent replication of influenza virus A/IND/5/05 and all six mice tested positive (see table 1 above). Vaccination with the inactivated whole virus vaccine NIBRG-14 adjuvanted with Stimune® not only prevented replication of the homologous strain influenza virus A/VN/1194/04 but also that of A/HK/156/97 and A/IND/5/05.

### Serology

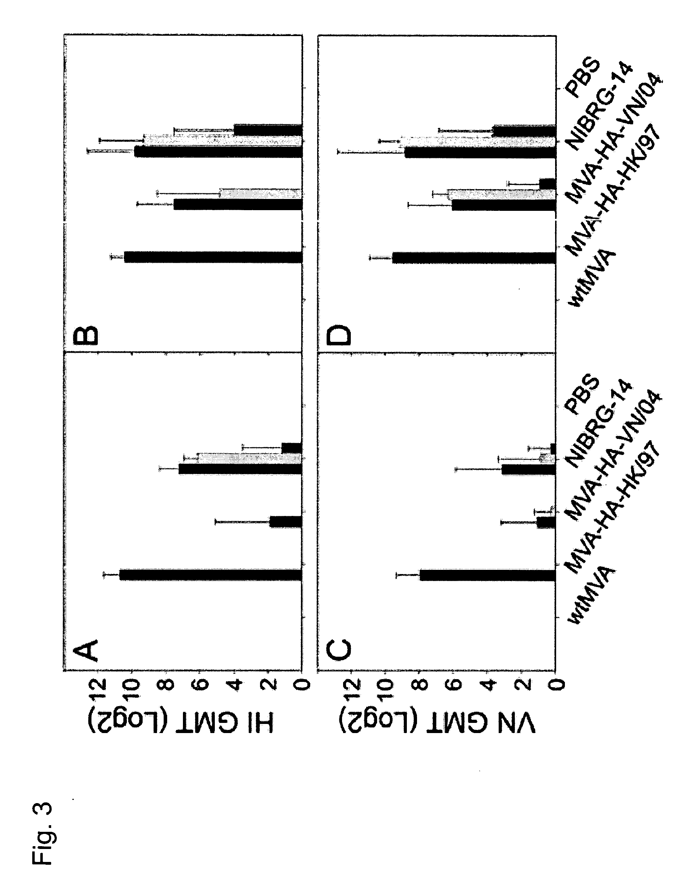

Upon a single vaccination with MVA-HA-HK/97 mice developed strong antibody responses against the homologous virus strain, however antibodies did not cross-react with the influenza virus strains A/VN/1194/04 or A/IND/5/05 as measured by HI and VN tests (Figure 3) (GMT of 1629 and 239 respectively). After the booster vaccination the homologous antibody titers in the HI- and VN-assay were 1370 and 744 GMT respectively. Again no cross-reaction was observed with the other H5N1 strains. The MVA-HA-VN/04 vaccine preparation was less immunogenic and after one vaccination only in one animal, antibodies against the homologous strain were detectable in the VN-assay. Upon a booster vaccination all animals responded and the GMT increased to 20 and 64 as measured by HI and VN assays respectively. The antibodies induced by MVA-HA-VN/04 vaccination cross-reacted with the H5N1 strain A/HK/156/97 and to a limited extent with the strain A/IND/5/05. The adjuvanted NIBRG-14 vaccine preparation, which was included in the experiments as a positive control induced robust antibody responses against the homologous A/VN/1194/04, which cross-reacted with the strain A/HK/156/97 and A/IND/5/05 both in the HI and VN assay.

### Pathological changes and virus replication in the lungs

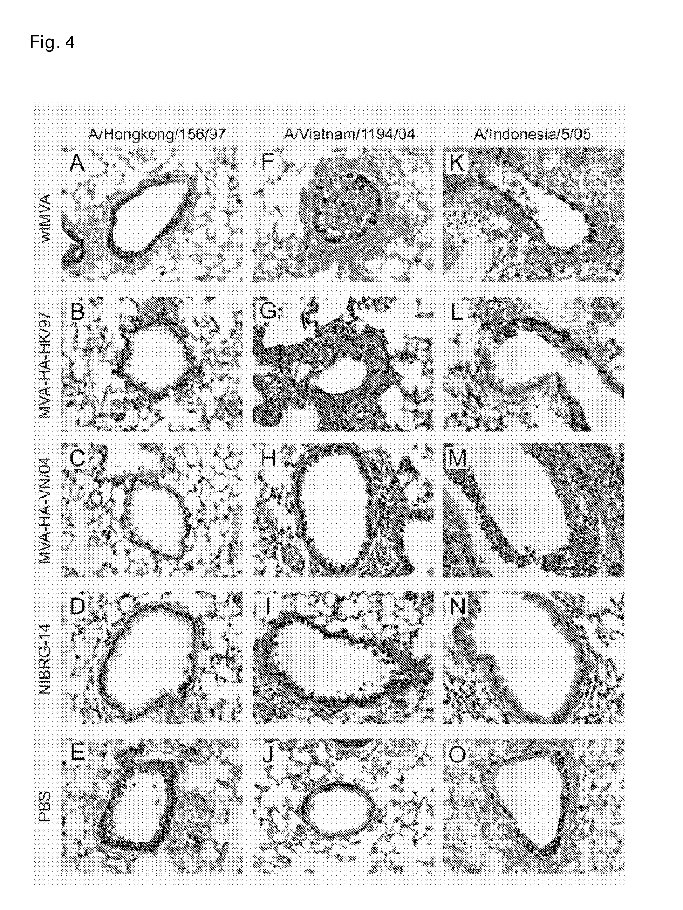

Four days after infection with either of the three HPAI viruses the mice were sacrificed and their lungs were inflated with formalin and examined histologically and using immunohistochemistry. Inoculation of wtMVA-and PBS-immunized mice with influenza virus A/Hongkong/156/97 resulted in a multifocal infection of the bronchiolar and alveolar epithelium, combined with a mild peribroncheal infiltrate and mild necrosis of the bronchiolar wall (Figure 4A, E). In the lungs of MVA-HA-HK/97 and MVA-HA-VN/04 immunized mice no virus on day 4 post infection was detected and no histopathological changes were observed (Figure 4B, C). Influenza virus A/VN/1194/04 infection led to stronger multifocal infection of the peribronchiolar and alveolar epithelium in both PBS and wtMVA immunized mice (Figure AF, J). Furthermore these mice suffered from moderate interstitial pneumonia based on loss of bronchiolar epithelium, strong peribroncheal infiltration and alveolar damage. Infection of the alveolar epithelium of MVA-HA-HK/97 immunized mice appeared to be less, however alveolar infiltration was more pronounced (Figure 4G). In the lungs of MVA-HA-VN/04 immunized mice no virus replication was detected and morphology was normal (Figure 4H). Infection with influenza virus A/IND/5/05 caused widespread infection of both bronchiolar and alveolar epithelium, in wtMVA, PBS and MVA-HA-HK/97 immunized mice (Figure 4K, L, O). Furthermore moderate infiltrate in both the bronchioles and alveoli was seen, next to loss of bronchiolar epithelium and the presence of intra-bronchiolar cell debri. MVA-HA-VN/04 immunized mice had a small number of peribronchiolar influenza virus A/IND/5/05 infected cells combined with moderate peribronchiolar infiltrate (Figure 4M). Mice immunized with adjuvanted NIBRG-14 had normal lungs, and no virus replication was seen after infection with either of the three influenza H5N1 viruses (Figure 4D, I, N).

### Figure captions

Fig.1
   Weight loss of mice intranassaly infected with 10³ TCID50 of influenza virus A/Hongkong/156/97 (A), A/Vietnam/1194/04 (B), or A/Indonesia/5/05 (C). Mean weight loss is expressed as the percentage of the original weight before infection. (*) indicates a statistically significant difference with (p < 0.05).
Fig.2
   Virus titers in organ tissue at day 4 after infection with either influenza virus A/Hongkong/156/97 (A), A/Vietnam/1194/04 (B), or A/Indonesia/5/05 (C). Results are shown for the wtMVA, MVA-HA-HK/97, MVA-HA-VN/04, Stimune®-adjuvanted NIBRG14 and PBS immunized mice. Titers were measured in: brain ( 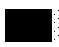 ), intestines ( 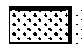 ), lungs ( 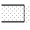 ) and spleen ( 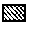 ) and presented as TCID50 per gram tissue (Log10). (*) Indicates that at least one animal tested positive for the indicated organ.
Fig.3
   Antibody responses induced by vaccination. Antibody titers against the three challenge viruses: influenza virus A/Hongkong/156/07 ( 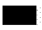 ), A/Vietnam/1194/04 ( 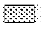 ) and A/Indonesia/5/05 ( 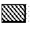 ) were measured by HI-assay (A+B). 28 days after the first immunization (A) and 28 days after the second immunization (B). Titers are presented as GMT (Log 2). Secondly antibody titers against the three different challenge viruses: influenza virus A/Hongkong/156/07 ( 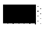 ), A/Vietnam/1194/04 ( 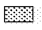 ) and A/Indonesia/5/05 ( 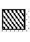 ) were measured by VN-assay (C+D). 28 days after the first immunization (C) and second immunization (D). Titers are presented as GMT (Log 2).
Fig.4
   Histopathology and immunohistochemistry of the bronchioles and alveoli in lungs of mice infected with either influenza virus A/Hongkong/157/97, A/Vietnam/1194/04 or A/Indonesia/5/05 as indicated. Influenza virus A/Hongkong/156/97 infection leads to virus positive cells in the bronchiolar wall of wtMVA (A) and PBS immunized mice (E), combined with mild peribronchiolar infiltrate, while in the lungs of MVA-HA-HK/97 (B), MVA-HA-VN/04 (C) and Stimune®-adjuvanted NIBRG-14 (D) immunized mice no virus was detected. Infection with influenza virus A/Vietnam/1194/04 results in virus positive cells in the bronchiolar walls of wtMVA (F), MVA-HA-HK/97 (G) and PBS (J) immunized mice, also combined with moderate peribronchiolar infiltrate (except for the PBS immunized mice). No virus replication or morphological changes were detected in MVA-HA-VN/04 (H) and Stimune®-adjuvanted NIBRG-14 immunized mice (I). Inoculation of influenza virus A/Indonesia/5/05 resulted in severe infection of the bronchioles of wtMVA (K), MVA-HA-HK/97 (L) and PBS (O) immunized mice, combined with moderate peribronchiolar infiltrate. Whereas only a minimal amount of cells in the bronchiolar wall of MVA-HA-VN/04 (M) immunized mice was infected, combined with moderate infiltrate. No virus was detected in the lungs of Stimune®-adjuvanted NIBRG-14 (N) immunized mice after infection with influenza virus A/Indonesia/5/05.
Fig. 5
   Construction of a recombinant MVA according to the invention

After introduction of the gene into the MVA transfer vector pIIIdHR under the control of the vaccinia promoter the gene was inserted into deletion III of the MVA genome by means of homologous recombination of the flanking regions (flank 1 and flank 2). Selection of recombinant viruses was performed by expression of the host range gene K1 L.

## Claims

1. Use of a recombinant modified vaccinia virus Ankara (MVA) comprising a heterologous nucleic acid for the production of a medicament in particular a vaccine, wherein the sequence of heterologous nucleic acid is from influenza A virus class H5 antigen.

2. Use according to claim 1, wherein the heterologous nucleic acid is incorporated into a non-essential site within the genome of MVA.

3. Use according to claim 2, wherein the heterologous nucleic acid is incorporated into the MVA genome at the site of deletion III.

4. Use according to claims 1 to 3, wherein the heterologous nucleic acid is under the control of a vaccinia virus-, or orthopoxvirus-, or poxvirus-specific promoter.

5. Use according to claims 1 to 4, wherein the sequence of the heterologous nucleic acid is selected from the group of H5N1, H5N3, H5N2, H5N7, H7N1,H7N7, H7N3, and H9N2 sequences.

6. Use according to claim 5, wherein the sequence of the heterologous nucleic acid is from H5N1 and is selected from the group of strains termed, A/Vietnam/1203/04, A/Vietnam/1 194/04, A/Vietnam/3046/04, A/Hongkong/156/97, A/HongKong/212/03, A/HongKong/213/03, A/Indonesia/5/05, A/Ck/Indonesia/BL03/03, A/Grey heron/HK/793.1/02, A/Black Headed Gull/HK/12.1/03, A/Dk/Vietnam/11/04, A/Ck/Vietnam/33/04, A/Ck/Vietnam/C-58/04, A/Dk/TH/D4AT/04, A/Gs/TH/G7CS/04, A/Dk/FJ/1734/O5, A/Ck/GX/463/06, A/Ck/HK/947/06.

7. Use according to claim 6, wherein the sequence of the heterologous nucleic acid of H5N1 is that of strain A/Vietnam/1 194/04, A/Indonesia/5/2005, A/Bar headed goose/Qinghai/1A/2005, A/Whooper swan/Mongolia/244/2005, A/turkey/Turkey/1/2005, and A/Anhui/1/2005.

8. Use according to claims 1 to 7, wherein the sequence of the heterologous nucleic acid encompasses the sequence or part of the sequence of the hemagglutinin gene from nucleotide 1 to 1500, from nucleotide 1 to 1100 or from nucleotide 40 to 1100.

9. Use according to claims 6 or 7, wherein the nucleic acid encompasses HA1 and HA2 subunits.

10. Use according to claims 6 to 9, wherein the nucleic acid encodes two or more sequences stemming from
a. different genes or parts thereof of a single H5N1 strain and/or,
b. different H5N1 strains.

11. Use according to claim 10, wherein the two or more sequences encode hemagglutinin or a part of hemagglutinin and stem from, A/Vietnam/1203/04, A/Vietnam/1 194/04, Vietnam/3046/04, A/Bar headed goose/Qinghai/1A/2005, A/Whooper swan/Mongolia/244/2005, A/turkey/Turkey/1/2005, and A/Anhui/1/2005, and/or A/IND/5/05.

12. Recombinant modified vaccinia virus Ankara (MVA) comprising a heterologous nucleic acid, wherein
a. the heterologous nucleic acid is incorporated into a non-essential site within the genome of MVA,
b. the heterologous nucleic acid is under the control of a vaccinia virus promoter, or orthopoxvirus promoter, or poxvirus-specific promoter and,
c. the heterologous nucleic acid is selected from the group of nucleic acids encoding a gene or a part of a gene from an influenza A virus class H5.

13. Recombinant modified vaccinia virus Ankara (MVA) according to claim 12, wherein the heterologous nucleic acid is selected from the group of H5N1, H5N3, H5N2, H5N7, H7N1,H7N7, H7N3, and H9N2 genome sequences.

14. Recombinant modified vaccinia virus Ankara (MVA) according to claim 13, wherein the heterologous nucleic acid is from H5N1 and is selected from the group of strains termed, A/Vietnam/1203/04, A/Vietnam/1194/04, A/Vietnam/3046/04, A/Hongkong/156/97, A/HongKong/212/03, A/HongKong/213/03, A/Indonesia/5/05 , A/Ck/Indonesia/BL03/03, A/Grey heron/HK/793.1/02, A/Black Headed Gull/HK/12.1/03, A/Dk/Vietnam/11/04, A/Ck/Vietnam/33/04, A/Ck/Vietnam/C-58/04, A/Dk/TH/D4AT/04, A/Gs/TH/G7CS/04, A/Dk/FJ/1734/O5, A/Ck/GX/463/06, A/Ck/HK/947/06.

15. Recombinant modified vaccinia virus Ankara (MVA) according to claim 14, wherein the heterologous nucleic acid of H5N1 is selected from the strains A/Vietnam/1 194/04, A/Indonesia/5/2005, A/Bar headed goose/Qinghai/1A/2005, A/Whooper swan/Mongolia/244/2005, A/turkey/Turkey/1/2005, and A/Anhui/1/2005.

16. Recombinant modified vaccinia virus Ankara (MVA) according to claim 15, wherein the sequence of the heterologous nucleic acid encompasses the sequence or part of the sequence of the hemagglutinin gene.

17. Recombinant modified vaccinia virus Ankara (MVA) according to claim 16, wherein the nucleic acid encompasses HA1 and HA2 subunits.

18. Recombinant modified vaccinia virus Ankara (MVA) according to claims 12 to 16, wherein the nucleic acid encodes two or more sequences stemming from
a. different genes or parts thereof of a single H5N1 strain and/or,
b. different H5N1 strains.

19. Recombinant modified vaccinia virus Ankara (MVA) according to claim 18, wherein the two or more sequences encode hemagglutinin or a part of hemagglutinin and are selected from sequences stemming from A/Vietnam/1203/04, A/Vietnam/1 194/04, A/Vietnam/3046/04, A/Hongkong/156/97, A/HongKong/212/03, A/HongKong/213/03, A/Indonesia/5/05, A/Ck/Indonesia/BL03/03, A/Grey heron/HK/793.1/02, A/Black Headed Gull/HK/12.1/03, A/Dk/Vietnam/11/04, A/Ck/Vietnam/33/04, A/Ck/Vietnam/C-58/04, A/Dk/TH/D4AT/04, A/Gs/TH/G7CS/04, A/Dk/FJ/1734/05, A/Ck/GX/463/06 and A/Ck/HK/947/06..

20. Nucleic acid encoding a modified vaccinia virus Ankara (MVA) according to any of claims 12 to 19.

21. Method of introducing a recombinant MVA according to any of claims 12 to 20 into a target cell comprising infection of the target cell with a recombinant MVA according to any of claims 16 to 25.

22. Method for immunization of a living animal body including a human said method comprising administering to said living animal body including a human in need thereof a therapeutically effective amount of the MVA according to claims 16 to 20.

23. Method according to claim 22, wherein the immunizing may be both prophylactic and/or therapeutic.


SEQUENCE LISTING

<110>  Paul-Ehrlich Institut
       Paul-Ehrlich-Institut

<120>  Recombinant modified vaccinia virus Ankara (MVA)-based vaccine
       for the avian flu

<130>  FBP18089

<160>  10

<170>  PatentIn version 3.3

<210>  1
<211>  1500
<212>  DNA
<213>  Avian influenza virus (H5N1)

<400>  1
atggagaaaa tagtgcttct ttttgcaata gtcagtcttg ttaaaagtga tcagatttgc      60

attggttacc atgcaaacaa ctcgacagag caggttgaca caataatgga aaagaacgtt     120

actgttacac atgcccaaga catactggaa aagacacaca atgggaagct ctgcgatcta     180

gatggagtga agcctctaat tttgagagat tgtagtgtag ctggatggct cctcggaaac     240

ccaatgtgtg acgaattcat caatgtgccg gaatggtctt acatagtgga gaaggccaat     300

ccagtcaatg acctctgtta cccaggggat ttcaatgact atgaagaatt gaaacaccta     360

ttgagcagaa taaaccattt tgagaaaatt cagatcatcc ccaaaagttc ttggtccagt     420

catgaagcct cattgggggt gagctcagca tgtccatacc agggaaagtc ctcctttttc     480

agaaatgtgg tatggcttat caaaaagaac agtacatacc caacaataaa gaggagctac     540

aataatacca accaagaaga tcttttggta ctgtggggga ttcaccatcc taatgatgcg     600

gcagagcaga caaagctcta tcaaaaccca accacctata tttccgttgg gacatcaaca     660

ctaaaccaga gattggtacc aagaatagct actagatcca aagtaaacgg gcaaagtgga     720

aggatggagt tcttctggac aattttaaaa ccgaatgatg caatcaactt cgagagtaat     780

ggaaatttca ttgctccaga atatgcatac aaaattgtca agaaagggga ctcaacaatt     840

atgaaaagtg aattggaata tggtaactgc aacaccaagt gtcaaactcc aatgggggcg     900

ataaactcta gcatgccatt ccacaatata caccctctca ccatcgggga atgccccaaa     960

tatgtgaaat caaacagatt agtccttgcg actgggctca gaaatagccc tcaaagagag    1020

agaagaagaa aaaagagagg attatttgga gctatagcag gttttataga gggaggatgg    1080

cagggaatgg tagatggttg gtatgggtac caccatagca acgagcaggg gagtgggtac    1140

gctgcagaca aagaatccac tcaaaaggca atagatggag tcaccaataa ggtcaactcg    1200

attattgaca aaatgaacac tcagtttgag gccgttggaa gggaatttaa caacttagaa    1260

aggagaatag agaatttaaa caagaagatg gaagacgggt tcctagatgt ctggacttat    1320

aatgctgaac ttctagttct catggaaaac gagagaactc tagactttca tgactcaaat   1380

gtcaagaacc tttacgacaa ggtccgacta cagcttaggg ataatgcaaa ggagctgggt   1440

aacggttgtt tcgagttcta tcataaatgt gataatgaat gtatggaaag tgtaagaaac   1500


<210>  2
<211>  1100
<212>  DNA
<213>  Avian influenza virus (H5N1)

<400>  2
atggagaaaa tagtgcttct ttttgcaata gtcagtcttg ttaaaagtga tcagatttgc     60

attggttacc atgcaaacaa ctcgacagag caggttgaca caataatgga aaagaacgtt    120

actgttacac atgcccaaga catactggaa aagacacaca atgggaagct ctgcgatcta    180

gatggagtga agcctctaat tttgagagat tgtagtgtag ctggatggct cctcggaaac    240

ccaatgtgtg acgaattcat caatgtgccg gaatggtctt acatagtgga gaaggccaat    300

ccagtcaatg acctctgtta cccaggggat ttcaatgact atgaagaatt gaaacaccta    360

ttgagcagaa taaaccattt tgagaaaatt cagatcatcc ccaaaagttc ttggtccagt    420

catgaagcct cattgggggt gagctcagca tgtccatacc agggaaagtc ctcctttttc    480

agaaatgtgg tatggcttat caaaaagaac agtacatacc caacaataaa gaggagctac    540

aataatacca accaagaaga tcttttggta ctgtggggga ttcaccatcc taatgatgcg    600

gcagagcaga caaagctcta tcaaaaccca accacctata tttccgttgg gacatcaaca    660

ctaaaccaga gattggtacc aagaatagct actagatcca aagtaaacgg gcaaagtgga    720

aggatggagt tcttctggac aattttaaaa ccgaatgatg caatcaactt cgagagtaat    780

ggaaatttca ttgctccaga atatgcatac aaaattgtca agaaagggga ctcaacaatt    840

atgaaaagtg aattggaata tggtaactgc aacaccaagt gtcaaactcc aatgggggcg    900

ataaactcta gcatgccatt ccacaatata caccctctca ccatcgggga atgccccaaa    960

tatgtgaaat caaacagatt agtccttgcg actgggctca gaaatagccc tcaaagagag   1020

agaagaagaa aaaagagagg attatttgga gctatagcag gttttataga gggaggatgg   1080

cagggaatgg tagatggttg                                               1100


<210>  3
<211>  1421
<212>  DNA
<213>  Avian influenza virus (H5N1)

<400>  3
atggagaaaa tagtgcttct ttttgcaata gtcagtcttg ttaaaagtga tcagatttgc     60

attggttacc atgcaaacaa ctcgacagag caggttgaca caataatgga aaagaacgtt    120

actgttacac atgcccaaga catactggaa aagacacaca atgggaagct ctgcgatcta 180

gatggagtga agcctctaat tttgagagat tgtagtgtag ctggatggct cctcggaaac 240

ccaatgtgtg acgaattcat caatgtgccg gaatggtctt acatagtgga gaaggccaat 300

ccagtcaatg acctctgtta cccaggggat ttcaatgact atgaagaatt gaaacaccta 360

gttaaaagtg atcagatttg cattggttac catgcaaaca actcgacaga gcaggttgac 420

acaataatgg aaaagaacgt tactgttaca catgcccaag acatactgga aaagacacac 480

aatgggaagc tctgcgatct agatggagtg aagcctctaa ttttgagaga ttgtagtgta 540

gctggatggc tcctcggaaa cccaatgtgt gacgaattca tcaatgtgcc ggaatggtct 600

tacatagtgg agaaggccaa tccagtcaat gacctctgtt acccagggga tttcaatgac 660

tatgaagaat tgaaacacct attgagcaga ataaaccatt ttgagaaaat tcagatcatc 720

cccaaaagtt cttggtccag tcatgaagcc tcattggggg tgagctcagc atgtccatac 780

cagggaaagt cctccttttt cagaaatgtg gtatggctta tcaaaaagaa cagtacatac 840

ccaacaataa agaggagcta caataatacc aaccaagaag atcttttggt actgtggggg 900

attcaccatc ctaatgatgc ggcagagcag acaaagctct atcaaaaccc aaccacctat 960

atttccgttg ggacatcaac actaaaccag agattggtac caagaatagc tactagatcc 1020

aaagtaaacg ggcaaagtgg aaggatggag ttcttctgga caattttaaa accgaatgat 1080

gcaatcaact tcgagagtaa tggaaatttc attgctccag aatatgcata caaaattgtc 1140

aagaaagggg actcaacaat tatgaaaagt gaattggaat atggtaactg caacaccaag 1200

tgtcaaactc caatgggggc gataaactct agcatgccat tccacaatat acaccctctc 1260

accatcgggg aatgccccaa atatgtgaaa tcaaacagat tagtccttgc gactgggctc 1320

agaaatagcc ctcaaagaga gagaagaaga aaaaagagag gattatttgg agctatagca 1380

ggttttatag agggaggatg gcagggaatg gtagatggtt g 1421

<210> 4
<211> 337
<212> PRT
<213> Avian influenza virus (H5N1)

<400> 4

Lys Ser Asp Gln Ile Cys Ile Gly Tyr His Ala Asn Asn Ser Thr Glu
1               5                   10                  15

Gln Val Asp Thr Ile Met Glu Lys Asn Val Thr Val Thr His Ala Gln
            20                  25                  30

Asp Ile Leu Glu Lys Thr His Asn Gly Lys Leu Cys Asp Leu Asp Gly
        35                  40                  45

Val Lys Pro Leu Ile Leu Arg Asp Cys Ser Val Ala Gly Trp Leu Leu
    50                  55                  60

Gly Asn Pro Met Cys Asp Glu Phe Ile Asn Val Pro Glu Trp Ser Tyr
65                  70                  75                  80

Ile Val Glu Lys Ala Asn Pro Val Asn Asp Leu Cys Tyr Pro Gly Asp
                85                  90                  95

Phe Asn Asp Tyr Glu Glu Leu Lys His Leu Leu Ser Arg Ile Asn His
            100                 105                 110

Phe Glu Lys Ile Gln Ile Ile Pro Lys Ser Ser Trp Ser Ser His Glu
        115                 120                 125

Ala Ser Leu Gly Val Ser Ser Ala Cys Pro Tyr Gln Gly Lys Ser Ser
    130                 135                 140

Phe Phe Arg Asn Val Val Trp Leu Ile Lys Lys Asn Ser Thr Tyr Pro
145                 150                 155                 160

Thr Ile Lys Arg Ser Tyr Asn Asn Thr Asn Gln Glu Asp Leu Leu Val
                165                 170                 175

Leu Trp Gly Ile His His Pro Asn Asp Ala Ala Glu Gln Thr Lys Leu
            180                 185                 190

Tyr Gln Asn Pro Thr Thr Tyr Ile Ser Val Gly Thr Ser Thr Leu Asn
        195                 200                 205

Gln Arg Leu Val Pro Arg Ile Ala Thr Arg Ser Lys Val Asn Gly Gln
    210                 215                 220

Ser Gly Arg Met Glu Phe Phe Trp Thr Ile Leu Lys Pro Asn Asp Ala
225                 230                 235                 240

Ile Asn Phe Glu Ser Asn Gly Asn Phe Ile Ala Pro Glu Tyr Ala Tyr
                245                 250                 255

Lys Ile Val Lys Lys Gly Asp Ser Thr Ile Met Lys Ser Glu Leu Glu
            260                 265                 270

Tyr Gly Asn Cys Asn Thr Lys Cys Gln Thr Pro Met Gly Ala Ile Asn
        275                 280                 285

Ser Ser Met Pro Phe His Asn Ile His Pro Leu Thr Ile Gly Glu Cys
    290                 295                 300

Pro Lys Tyr Val Lys Ser Asn Arg Leu Val Leu Ala Thr Gly Leu Arg
305                 310                 315                 320

Asn Ser Pro Gln Arg Glu Arg Arg Arg Lys Lys Arg Gly Leu Phe Gly
                325                 330                 335

Ala

<210>  5
<211>  149
<212>  PRT
<213>  Avian influenza virus (H5N1)

<400>  5

Ile Ala Gly Phe Ile Glu Gly Gly Trp Gln Gly Met Val Asp Gly Trp
1               5                   10                  15

Tyr Gly Tyr His His Ser Asn Glu Gln Gly Ser Gly Tyr Ala Ala Asp
            20                  25                  30

Lys Glu Ser Thr Gln Lys Ala Ile Asp Gly Val Thr Asn Lys Val Asn
        35                  40                  45

Ser Ile Ile Asp Lys Met Asn Thr Gln Phe Glu Ala Val Gly Arg Glu
    50                  55                  60

Phe Asn Asn Leu Glu Arg Arg Ile Glu Asn Leu Asn Lys Lys Met Glu
65                  70                  75                  80

Asp Gly Phe Leu Asp Val Trp Thr Tyr Asn Ala Glu Leu Leu Val Leu
                85                  90                  95

Met Glu Asn Glu Arg Thr Leu Asp Phe His Asp Ser Asn Val Lys Asn
            100                 105                 110

Leu Tyr Asp Lys Val Arg Leu Gln Leu Arg Asp Asn Ala Lys Glu Leu
        115                 120                 125

Gly Asn Gly Cys Phe Glu Phe Tyr His Lys Cys Asp Asn Glu Cys Met
    130                 135                 140

Glu Ser Val Arg Asn
145

<210>  6
<211>  41

<212>  PRT
<213>  Avian influenza virus (H5N1)

<400>  6

Leu Leu Ser Arg Ile Asn His Phe Glu Lys Ile Gln Ile Ile Pro Lys
1               5                   10                  15

Ser Ser Trp Ser Ser His Glu Ala Ser Leu Gly Val Ser Ser Ala Cys
            20                  25                  30

Pro Tyr Gln Gly Lys Ser Ser Phe Phe
        35                  40

<210>  7
<211>  111
<212>  PRT
<213>  Avian influenza virus (H5N1)

<400>  7

Leu Leu Ser Arg Ile Asn His Phe Glu Lys Ile Gln Ile Ile Pro Lys
1               5                   10                  15

Ser Ser Trp Ser Ser His Glu Ala Ser Leu Gly Val Ser Ser Ala Cys
            20                  25                  30

Pro Tyr Gln Gly Lys Ser Ser Phe Phe Arg Asn Val Val Trp Leu Ile
        35                  40                  45

Lys Lys Asn Ser Thr Tyr Pro Thr Ile Lys Arg Ser Tyr Asn Asn Thr
    50                  55                  60

Asn Gln Glu Asp Leu Leu Val Leu Trp Gly Ile His His Pro Asn Asp
65                  70                  75                  80

Ala Ala Glu Gln Thr Lys Leu Tyr Gln Asn Pro Thr Thr Tyr Ile Ser
                85                  90                  95

Val Gly Thr Ser Thr Leu Asn Gln Arg Leu Val Pro Arg Ile Ala
            100                 105                 110

<210>  8
<211>  51
<212>  PRT
<213>  Avian influenza virus (H5N1)

<400>  8

Tyr Asn Asn Thr Asn Gln Glu Asp Leu Leu Val Leu Trp Gly Ile His
1               5                   10                  15

His Pro Asn Asp Ala Ala Glu Gln Thr Lys Leu Tyr Gln Asn Pro Thr
            20                  25                  30

Thr Tyr Ile Ser Val Gly Thr Ser Thr Leu Asn Gln Arg Leu Val Pro
        35                  40                  45

Arg Ile Ala
    50

<210>  9
<211>  12
<212>  DNA
<213>  Artificial

<220>
<223>  primer

<400>  9
agcaaaagca gg                                                          12

<210>  10
<211>  351
<212>  PRT
<213>  Avian influenza virus

<400>  10

Met Glu Lys Ile Val Leu Leu Phe Ala Ile Val Ser Leu Val Lys Ser
1               5                   10                  15

Asp Gln Ile Cys Ile Gly Tyr His Ala Asn Asn Ser Thr Glu Gln Val
            20                  25                  30

Asp Thr Ile Met Glu Lys Asn Val Thr Val Thr His Ala Gln Asp Ile
        35                  40                  45

Leu Glu Lys Thr His Asn Gly Lys Leu Cys Asp Leu Asp Gly Val Lys
    50                  55                  60

Pro Leu Ile Leu Arg Asp Cys Ser Val Ala Gly Trp Leu Leu Gly Asn
65                  70                  75                  80

Pro Met Cys Asp Glu Phe Ile Asn Val Pro Glu Trp Ser Tyr Ile Val
                85                  90                  95

Glu Lys Ala Asn Pro Val Asn Asp Leu Cys Tyr Pro Gly Asp Phe Asn
            100                 105                 110

Asp Tyr Glu Glu Leu Lys His Leu Leu Ser Arg Ile Asn His Phe Glu
        115                 120                 125

Lys Ile Gln Ile Ile Pro Lys Ser Ser Trp Ser Ser His Glu Ala Ser
    130                 135                 140

Leu Gly Val Ser Ser Ala Cys Pro Tyr Gln Gly Lys Ser Ser Phe Phe
145                 150                 155                 160

Arg Asn Val Val Trp Leu Ile Lys Lys Asn Ser Thr Tyr Pro Thr Ile
                165                 170                 175

Lys Arg Ser Tyr Asn Asn Thr Asn Gln Glu Asp Leu Leu Val Leu Trp
            180                 185                 190

Gly Ile His His Pro Asn Asp Ala Ala Glu Gln Thr Lys Leu Tyr Gln
        195                 200                 205

Asn Pro Thr Thr Tyr Ile Ser Val Gly Thr Ser Thr Leu Asn Gln Arg
    210                 215                 220

Leu Val Pro Arg Ile Ala Thr Arg Ser Lys Val Asn Gly Gln Ser Gly
225                 230                 235                 240

Arg Met Glu Phe Phe Trp Thr Ile Leu Lys Pro Asn Asp Ala Ile Asn
                245                 250                 255

Phe Glu Ser Asn Gly Asn Phe Ile Ala Pro Glu Tyr Ala Tyr Lys Ile
            260                 265                 270

Val Lys Lys Gly Asp Ser Thr Ile Met Lys Ser Glu Leu Glu Tyr Gly
        275                 280                 285

Asn Cys Asn Thr Lys Cys Gln Thr Pro Met Gly Ala Ile Asn Ser Ser
    290                 295                 300

Met Pro Phe His Asn Ile His Pro Leu Thr Ile Gly Glu Cys Pro Lys
305                 310                 315                 320

Tyr Val Lys Ser Asn Arg Leu Val Leu Ala Thr Gly Leu Arg Asn Ser
                325                 330                 335

Pro Gln Arg Glu Arg Arg Arg Lys Lys Arg Gly Leu Phe Gly Ala
            340                 345                 350